# EUROPEAN PATENT APPLICATION

(11) **EP 2 014 284 A1**
(43) Date of publication of application: **14.01.2009**
(21) Application number: 07110419.4
(22) Date of filing: 15.06.2007
(51) Int. Cl.: A61K 31/136, A61K 31/55, A61K 38/06, A61P 9/02, A61P 9/12, A61P 19/02, A61P 29/00

(54) **Pharmaceutical compositions and uses**

(71) Applicant: NOVARTIS AG, 4002 Basel (CH)
(72) Inventor: The designation of the inventor has not yet been filed
(74) Representative: Leon, Susanna Iris

(57) **Abstract**

Pharmaceutical compositions, combinations and uses comprising a COX-2 inhibitor and an antihypertensive.

## Description

This invention relates to COX-2 inhibitors, in particular to combined use of COX-2 inhibitors with anti-hypertensive agents, to compositions containing such combinations and to the use of COX-2 inhibitors in patients with hypertension.

Independent research shows that even small elevations in blood pressure can contribute to an increased risk of cardiovascular events. NSAIDs (non-steroidal anti-inflammatory drugs), including some COX-2 inhibitors, have been associated with raised blood pressure, and this effect has been thought to be in part responsible for the increased risk of cardiovascular disease associated with this class of medications. Indeed, the use of NSAIDs is even contraindicated for patients suffering from or at risk of hypertension.

The present invention is based on the entirely unexpected finding that COX-2 inhibitors, particularly lumiracoxib, when administered to a patient with controlled hypertension, have a significantly improved blood pressure profile compared to other NSAIDs. Unlike other NSAIDs which have been shown to raise blood pressure, lumiracoxib has been shown to reduce blood pressure when administered to patients already taking an antihypertensive.

Accordingly the invention in one aspect provides a combination comprising a COX-2 inhibitor and an antihypertensive for the treatment of hypertension.

In a further aspect the invention provides a combination comprising a COX-2 inhibitor and an antihypertensive for the treatment of a COX-2 dependent disorder.

The combinations and compositions of the present invention may be provided as a fixed or free combination, for simultaneous, separate and/or sequential administration, for example as a single dosage form such as a pill, capsule or liquid containing both active agents, or as a co-pack comprising the agents separately, or as separate individual presentations of the active agents.

In a further aspect the invention provides a pharmaceutical composition for the treatment of a COX-2 dependent disorder, the composition comprising a COX-2 inhibitor and an antihypertensive.

In a further aspect the invention provides a pharmaceutical composition for the treatment of hypertension, the composition comprising a COX-2 inhibitor and an antihypertensive.

In a further aspect the invention provides a pharmaceutical composition for the treatment of pain in a hypertensive patient with osteoarthritis, the composition comprising a COX-2 inhibitor and an antihypertensive.

Further the invention provides:
(i) the use of a COX-2 inhibitor for the preparation of a medicament for use in combination with an antihypertensive for treatment of pain in a hypertensive patient.
(ii) the use of an antihypertensive for the preparation of a medicament for use in combination a COX-2 inhibitor for treatment of pain in a hypertensive patient;
(iii) the use of a COX-2 inhibitor for the preparation of a medicament for use in combination with an antihypertensive for treatment of hypertension;
(iv) the use of an antihypertensive for the preparation of a medicament for use in combination a COX-2 inhibitor for treatment of hypertension;
(v) a method of treating a patient suffering from pain comprising administering to the patient an effective amount of a COX-2 inhibitor and an antihypertensive, the COX-2 inhibitor and antihypertensive being administered simultaneously, separately or sequentially;
(vi) a method of treating or preventing hypertension in a patient in need thereof comprising administering to the patient an effective amount of a COX-2 inhibitor and an antihypertensive, the COX-2 inhibitor and antihypertensive being administered simultaneously, separately or sequentially;
(vii) a method of reducing blood pressure in a hypertensive patient comprising administering to the patient an effective amount of a COX-2 inhibitor and an antihypertensive, the COX-2 inhibitor and antihypertensive being administered simultaneously, separately or sequentially;
(viii) a package comprising a COX-2 inhibitor together with instructions for use in combination with an antihypertensive for treatment of pain in a patient suffering from hypertension;
(ix) a package comprising a COX-2 inhibitor and an antihypertensive together with instructions for use for treatment of pain in a patient suffering from hypertension;
(x) the use of a COX-2 inhibitor in the manufacture of a medicament for the treatment of pain in a patient with osteoarthritis and controlled hypertension.
(xi) the use of a COX-2 inhibitor in the manufacture of a medicament for the treatment of pain in a patient with osteoarthritis, wherein the patient is also receiving an antihypertensive agent for the treatment of hypertension.

Pain in general may be treated in accordance with the present invention including both nociceptive and inflammatory pain. In particular the combination treatment of the invention may be used for the treastment of musculoskeletal pain, especially lower back pain. More particularly the pain referred to herein refers to pain related to osteoarthritis.

In the present description the terms "treatment" or "treat" refer to both prophylactic or preventative treatment as well as curative or disease modifying treatment, including treatment of patients at risk of suffering pain as well as patients who are already suffering pain.

The COX-2 inhibitors used in the pharmaceutical compositions and treatment methods of the present invention are typically those which have an lC₅₀ for COX-2 inhibition less than about 2µM and an IC₅₀ for COX-1 inhibition greater than about 5µM, e.g. when measured in the assays described by Brideau et al.in Inflamm. Res. 45:68-74 (1996). Preferably the COX-2 inhibitor has a selectivity ratio of at least 10, more preferably at least 40, for COX-2 inhibition over COX-1 inhibition.

Thus, for example, suitable COX-2 inhibitors for use in the invention may include the following compounds or derivatives thereof or a pharmaceutically acceptable salt thereof, or any hydrate thereof: rofecoxib, etoricoxib, celecoxib, valdecoxib, parecoxib, or a 5-alkyl-2-arylaminophenylacetic acid derivative COX-2 inhibitor, e.g. of formula V as defined below.

Alternative classes of COX-2 inhibitor compounds for use in the invention include those described in US Patent No. 6,136,804 (Merck).

COX-2 inhibitors of formula V are particularly preferred for use in the present invention.

Thus in preferred embodiments the COX-2 inhibitor for use in the present invention comprises a compound of formula V 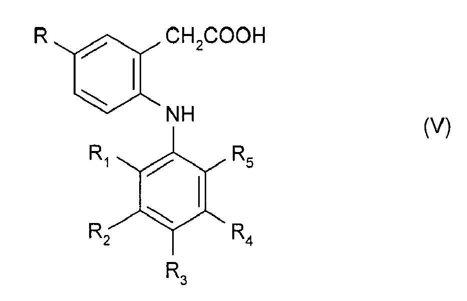
- wherein: R is methyl or ethyl;
R₁ is chloro or fluoro;
R₂ is hydrogen or fluoro;
R₃ is hydrogen, fluoro, chloro, methyl, ethyl, methoxy, ethoxy or hydroxy;
R₄ is hydrogen or fluoro; and
R₅ is chloro, fluoro, trifluoromethyl or methyl.

Above and elsewhere in the present description the terms "a benzothiadiazole derivative" and "COX-2 inhibitor" include, as appropriate, pharmaceutically acceptable salts and esters thereof.

Particularly preferred compounds of formula V are those wherein R is methyl or ethyl; R₁ is chloro or fluoro; R₂ is hydrogen; R₃ is hydrogen, fluoro, chloro, methyl or hydroxy; R₄ is hydrogen; and R₅ is chloro, fluoro or methyl; pharmaceutically acceptable salts thereof; and pharmaceutically acceptable esters thereof.

A particularly preferred embodiment relates to the compounds of formula V wherein R is methyl or ethyl; R₁ is fluoro; R₂ is hydrogen; R₃ is hydrogen, fluoro or hydroxy; R₄ is hydrogen; and R₅ is chloro; pharmaceutically acceptable salts thereof; and pharmaceutically acceptable prodrug esters thereof.

Another particularly preferred embodiment of the invention relates to compounds of formula V wherein R is ethyl or methyl; R₁ is fluoro; R₂ is hydrogen or fluoro; R₃ is hydrogen, fluoro, ethoxy or hydroxy; R₄ is hydrogen or fluoro; and R₅ is chloro, fluoro or methyl; pharmaceutically acceptable salts thereof; and pharmaceutically acceptable prodrug esters thereof.

Further are said compounds wherein R is methyl or ethyl; R₁ is fluoro; R₂-R₄ are hydrogen or fluoro; and R₅ is chloro or fluoro; pharmaceutically acceptable salts thereof; and pharmaceutically acceptable prodrug esters thereof.

A further embodiment of the invention relates to the compounds of formula V wherein R is methyl or ethyl; R₁ is fluoro; R₂ is fluoro; R₃ is hydrogen, ethoxy or hydroxy; R₄ is fluoro; and R₅ is fluoro; pharmaceutically acceptable salts thereof; and pharmaceutically acceptable prodrug esters thereof.

Another embodiment of the invention relates to the compounds of formula V wherein R is methyl; R₁ is fluoro; R₂ is hydrogen; R₃ is hydrogen or fluoro; R₄ is hydrogen; and R₅ is chloro; pharmaceutically acceptable salts thereof; and pharmaceutically acceptable prodrug esters thereof.

### Particularly preferred embodiments of the invention relate to compounds of formula V

(a) wherein R is methyl; R₁ is fluoro; R₂ is hydrogen; R₃ is hydrogen; R₄ is hydrogen; and R₅ is chloro; pharmaceutically acceptable salts thereof; and pharmaceutically acceptable prodrug esters thereof;
(b) wherein R is methyl; R₁ is fluoro; R₂ is hydrogen; R₃ is fluoro; R₄ is hydrogen; and R₅ is chloro; pharmaceutically acceptable salts thereof; and pharmaceutically acceptable prodrug esters thereof;
(c) wherein R is ethyl; R₁ is fluoro; R₂ is fluoro; R₃ is hydrogen; R₄ is fluoro; and R₅ is fluoro; pharmaceutically acceptable salts thereof; and pharmaceutically acceptable prodrug esters thereof; and
(d) wherein R is ethyl; R₁ is chloro; R₂ is hydrogen; R₃ is chloro; R₄ is hydrogen; and R₅ is methyl; pharmaceutically acceptable salts thereof; and pharmaceutically acceptable prodrug esters thereof.

Most preferably the COX-2 inhibitor of formula V is 5-methyl-2-(2'-chloro-6'-fluoroanilino)phenylacetic acid (lumiracoxib), or a salt or ester thereof.

In an alternatively preferred embodiment the COX-2 inhibitor is a compound of formula I: 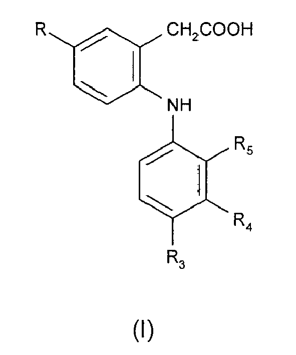 wherein
R is methyl or ethyl;
R₃ is halo or C₁-C₆ alkyl;
R₄ is C₁-C₆ alkyl;
R₅ is halo;
the above mentioned C₁-C₆ alkyl at R₃ and R₄ being optionally substituted by one or more halo groups;
pharmaceutically acceptable salts thereof; and pharmaceutically acceptable esters thereof.

Preferably in compounds of formula (I), R₃ is halo, methyl or ethyl. More preferably it is halo. Alternatively preferably it is chloro.

Preferably in compounds of formula (I), R₄ is C₁-C₃ alkyl, optionally substituted by one or more halo groups. More preferably it is methyl, ethyl, isopropyl or trifluoromethyl. Yet more preferably it is methyl. Alternatively preferably it is trifluoromethyl.

Preferably in compounds of formula (I), R₅ is chloro or fluoro.

Yet more preferably both R₃ and R₅ are independently selected from chloro and fluoro.

Preferably the compound is selected from the following list of compounds:
5-methyl-2-(2',4'-dichloro-3'-methylanilino)phenylacetic acid
5-ethyl-2-(2',4'-dichloro-3'-methylanilino)phenylacetic acid
5-methyl-2-(2'-fluoro-3'-trifluoromethyl-4'-ethylanilino)phenylacetic acid
5-ethyl-2-(2'-fluoro-3'-trifluoromethyl-4'-ethylanilino)phenylacetic acid
5-methyl-2-(2'-fluoro-3'-trifluoromethyl-4'-methylanilino)phenylacetic acid
5-ethyl-2-(2'-fluoro-3'-trifluoromethyl-4'-methylanilino)phenylacetic acid
5-methyl-2-(2'-fluoro-3'-methyl-4'chloroanilino)phenylacetic acid
5-ethyl-2-(2'-fluoro-3'-methyl-4'chloroanilino)phenylacetic acid
5-methyl-2-(2'-fluoro-3'-ethyl-4'chloroanilino)phenylacetic acid
5-ethyl-2-(2'-fluoro-3'-ethyl-4'chloroanilino)phenylacetic acid
5-methyl-2-(2',4'-dichloro-3'-ethylanilino)phenylacetic acid
5-ethyl-2-(2',4'-dichloro-3'-ethylanilino)phenylacetic acid
5-ethyl-2-(2'-fluoro-3'-isopropyl-4'chloroanilino)phenylacetic acid
5-Methyl-2-(2',4'-dichloro -3'-methylanilino)phenylacetic acid diethylamine salt sodium 5-Methyl-2-(2',4'-dichloro -3'-methylanilino)phenylacetate
5-Methyl-2-(2',4'-dichloro -3'-methylanilino)phenylacetic acid tromethamine salt calcium 5-Methyl-2-(2',4'-dichloro -3'-methylanilino)phenylacetate monohydrate Lysine 5-Methyl-2-(2',4'-dichloro -3'-methylanilino)phenylacetate monohydrate choline 5-Methyl-2-(2',4'-dichloro -3'-methylanilino)phenylacetate monohydrate Potassium 5-Methyl-2-(2',4'-dichloro -3'-methylanilino)phenylacetate.

Pharmaceutically acceptable prodrug esters of the compounds of formula V or I are ester derivatives which are convertible by solvolysis or under physiological conditions to the free carboxylic acids of formula V. Such esters are e.g. lower alkyl esters (such as the methyl or ethyl ester), carboxy-lower alkyl esters such as the carboxymethyl ester, nitrooxy-lower alkyl esters (such as the 4-nitrooxybutyl ester), and the like. Preferred prodrugs are the compounds of formula la 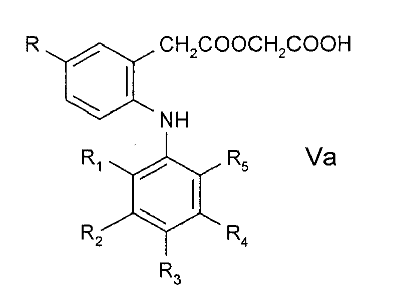 wherein R and R₁-R₅ have meaning as defined hereinabove for compounds of formula V and I; and pharmaceutically acceptable salts thereof.

Compounds of formula V, I and Va and their synthesis are described in published international patent applications Nos. WO 99/11605 and WO 01/23346, the teachings of which are incorporated herein by reference.

The antihypertensive employed in the present invention may be an anti-hypertensive agent selected from the following: loop diuretics such as ethacrynic acid, furosemide and torsemide; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase **(NEP) inhibitors** such as candoxatril or sinorphan; **ACE/NEP** inhibitors such as omapatrilat, sampatrilat and fasidotril; **angiotensin II antagonists** (AT₁-receptor antagonist) such as candesartan, eprosartan, irbesartan, losartan, telmisartan and valsartan, in particular valsartan; renin inhibitors such as ditekiren, zankiren, terlakiren, aliskiren, RO 66-1132 and RO-66-1168 - especially preferred is the compound of formula 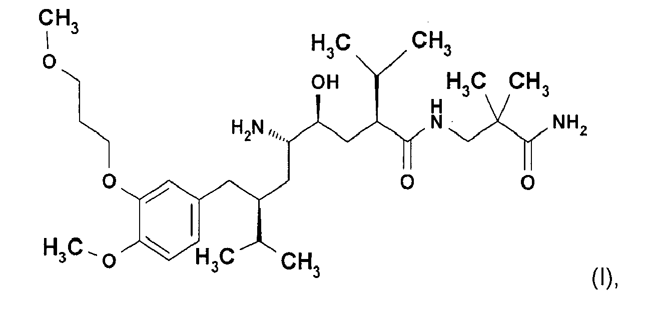 chemically defined as 2(S),4(S),5(S),7(S)-N-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide (generic name: aliskiren), specifically disclosed in EP 678503 A, or a pharmaceutically acceptable salt, especially the hemi-fumarate, thereof;
**β-adrenergic receptor** blockers such as acebutolol, atenolol, betaxolol, bisoprolol, metoprolol, nadolol, propranolol, sotalol and timolol; inotropic agents such as digoxin, dobutamine and milrinone; **calcium channel blockers** such as amlodipine, amlodipine besylate, bepridil, diltiazem, felodipine, nicardipine, nimodipine, nifedipine, nisoldipine and verapamil; aldosterone receptor antagonists such as eplerenone; aldosterone synthase inhibitor such as anastrazole , fadrozole or (+)-enantiomer of fadrozole; a beta blocker is metoprolol; alpha-adrenergic receptor blocker such as doxazosin, amosulalol,
dapiprazole, fenspiride, labetolol, indoramin, nicergoline, prazosin, tamsulosine, tolazoline, trimazosin, yohimbine; endothelin receptor antagonist such as bosentan; a diuretic such as hydrochlorothiazide ;or a **dual angiotensin converting enzyme/neutral endopetidase (ACE/NEP)** inhibitor such as omapatrilat,
Vasodilators such as cerebral vasodilators, coronary vasodilators and peripheral vasodilators e.g. bencyclane; cinnarizine; citicoline, cyclandelate, ciclonicate, diisopropylamine dichloroacetate, eburnamonine, fasudil, fenoxedil, flunarizine, ibudilast, ifenprodil, lomerizine, nafronyl, nicametate, nicergoline, nimodipine, papaverine, pentifylline, tinofedrine, vincamine, vinpocetine, and viquidil, Coronary vasodilators such as amotriphene, bendazol, benfurodil hemisuccinate, benziodarone, chloracizine, chromonar, clobenfural, clonitrate, cloricromen, dilazep, dipyridamole, droprenilamine, efloxate, erythrityl tetranitrate, etafenone, fendiline, floredil, ganglefene, hexestrol, hexobendine, itramin tosylate, khellin, lidoflazine, mannitol hexanitrate, medibazine, nitroglycerin; pentaerythritol pentrinitrol, perhexilline, pimefylline, prenylamine propatyl nitrate, trapidil, tricromyl, trimetazidine, trolnitrate phosphate and visnadine, Peripheral vasodilators such as aluminum nicotinate, bamethan, bencyclane, betahistine, bradykinin, brovincamine, bufeniode, buflomedil, butalamine, cetiedil, ciclonicate, cinepezide, cinnarizine, cyclandelate, diisopropylamine dichloroacetate, eledoisin, fenoxedil, flunarizine, hepronicate, ifenprodil, iloprost, inositol niacinate, isoxsuprine, kallidin, kallikrein, moxisylyte, nafronyl, nicametate, nicergoline, nicofuranose, nylidrin, pentifylline, pentoxifylline, piribedil, prostaglandin E, suloctidil, tolazoline, and xanthinol niacinate, diuretics including diuretic benzothiadiazine derivatives, diuretic organomercurials, diuretic purines, diuretic steroids, diuretic sulfonamide derivatives, diuretic uracils and other diuretics such as amanozine, amiloride, arbutin, chlorazanil, ethacrynic acid, which etozolin, hydracarbazine, isosorbide, mannitol; metochalcone, muzolimine, perhexiline, ticrynafen, triamterene and urea, althiazide, bendroflumethiazide; benzthiazide, benzylhydrochlorothiazide, buthiazide, chlorothiazide, chlorthalidone, cyclopenthiazide, cyclothiazide, epithiazide, ethiazide, fenquizone, indapamide, hydrochlorothiazide, hydroflumethiazide, methyclothiazide, meticrane, metolazone, paraflutizide, polythiazide, quinethazone, teclothiazide and trichlormethiazide, acetazolamide, ambuside, azosemide, bumetanide, butazolamide, chloraminophenamide, clofenamide, clopamide, clorexolone, disulfamide, ethoxolamide, furosemide, mefruside, methazolamide, piretanide, torasemide, tripamide, and xipamide,
or in any case a pharmaceutically accepted salt thereof.

The class of AT₁ receptor antagonists comprises compounds having differing structural features, essentially preferred are the non-peptidic ones. For example, mention may be made of the compounds that are selected from the group consisting of valsartan (cf. EP 443983), losartan (cf. EP253310), candesartan (cf. 459136), eprosartan (cf. EP 403159), irbesartan (cf. EP454511), olmesartan (cf. EP 503785), tasosartan (cf. EP539086), telmisartan (cf. EP 522314), saprisartan, saprisartan, the compound with the designation E-1477 of the following formula 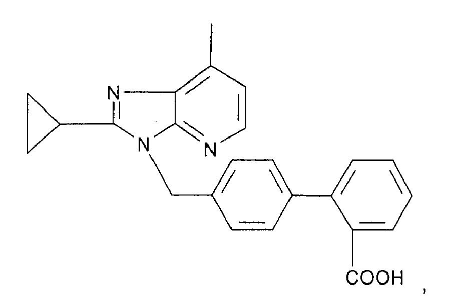 the compound with the designation SC-52458 of the following formula 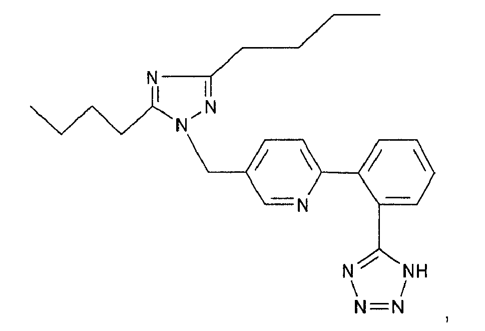 and the compound with the designation the compound ZD-8731 of the following formula 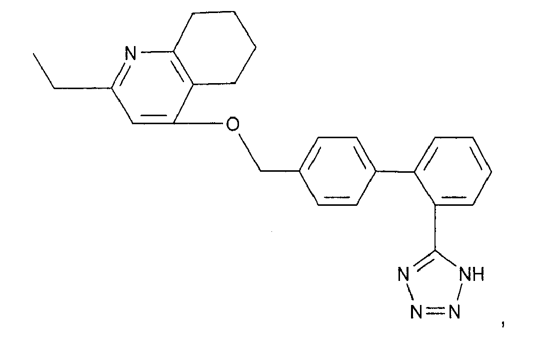 or, in each case, a pharmaceutically acceptable salt thereof.

Preferred AT₁-receptor antagonist are those agents that have been marketed, most preferred is valsartan or a pharmaceutically acceptable salt thereof.

In a particularly preferred embodiment of the invention the antihypertensive is an ACE inhibitor.

The class of ACE inhibitors which may be employed in the present invention comprises compounds having differing structural features. For example, mention may be made of the compounds which are selected from the group consisting alacepril, benazepril, benazeprilat, captopril, ceronapril, cilazapril, delapril, enalapril, enaprilat, fosinopril, imidapril, lisinopril, moveltopril, perindopril, quinapril, ramipril, spirapril, temocapril, and trandolapril, or, in each case, a pharmaceutically acceptable salt thereof.

Preferred ACE inhibitors are those agents that have been marketed; most preferred are benazepril and enalapril.

The structure of the therapeutic agents identified by code numbers, generic or trade names may be taken from the actual edition of the standard compendium "The Merck Index" or from databases, e.g., Patents International (e.g. IMS World Publications).

The Agents of the Invention, i.e. the COX-2 inhibitor and the antihypertensive are preferably used in the form of pharmaceutical preparations that contain the relevant therapeutically effective amount of of each active ingredient (either separately or in combination) optionally together with or in admixture with inorganic or organic, solid or liquid, pharmaceutically acceptable carriers which are suitable for administration. The Agents of the Invention may be present in the same pharmaceutical compositions, though are preferably in separate pharmaceutical compositions. Thus the active ingredients may be administered at the same time (e.g. simultaneously) or at different times (e.g. sequentially) and over different periods of time, which may be separate from one another or overlapping.

The pharmaceutical compositions may be, for example, compositions for enteral, such as oral, rectal, aerosol inhalation or nasal administration, compositions for parenteral, such as intravenous or subcutaneous administration, or compositions for transdermal administration (e.g. passive or iontophoretic).

The particular mode of administration and the dosage may be selected by the attending physician taking into account the particulars of the patient, especially age, weight, life style, activity level, and disease state as appropriate

Preferably, both the COX-2 inhibitor and antihypertensive pharmaceutical compositions are adapted for oral or parenteral (especially oral) administration. Intravenous and oral, first and foremost oral, adminstration is considered to be of particular importance. Preferably the COX-2 inhibitor active ingredient is in oral form.

The dosage of COX-2 inhibitor administered is dependent on the species of warm-blooded animal (mammal), the body weight, age and individual condition, and on the form of administration. A unit dosage for oral administration to a mammal of about 50 to 70 kg may contain between about 5 and 1500 mg, e.g. from 100-1000 mg, preferably 50-400 mg of the active ingredient.

COX-2 inhibitor formulations in single dose unit form contain preferably from about 1% to about 90%, and formulations not in single dose unit form contain preferably from about 20% to about 70%, of the active ingredient. Single dose unit forms such as capsules, tablets or dragées contain e.g. from about 1 mg to about 1500mg of the active ingredient. Lumiracoxib is preferably administered as a single dose of 50-400mg, preferably once or twice a day. Preferably, for example, 100mg of lumiracoxib is administered as a once daily dose.

Similarly the dosage of antihypertensive administered is dependent on the species of warm-blooded animal (mammal), the body weight, age and individual condition, and on the form of administration. In general, the daily dosage of antihypertensive varies between about 0.01 mg/kg and about 100 mg/kg. Suitable unit dosage forms, such as dragées, tablets or suppositories, preferably contain from about 10 to about 400mg of antihypertensive. Dosage units for oral administration preferably contain between 10% and 90% by weight of antihypertensive.

Preferred dosage unit forms of renin inhibitors are, for example, tablets or capsules comprising e.g. from about 5 mg to about 500 mg, preferably, when using aliskiren, for example, 50 to 250 mg (equivalent to the free acid) of aliskiren, for example, administered once a day.

Valsartan, as a representative of the class of AT₁-receptor antagonists, will be supplied in the form of suitable dosage unit form, for example, a capsule or tablet, and comprising a therapeutically effective amount, e.g. from about 20 to about 320 mg, of valsartan which may be applied to patients. The application of the active ingredient may occur up to three times a day, starting e.g. with a daily dose of 20 mg or 40 mg of valsartan, increasing via 80 mg daily and further to 160 mg daily up to 320 mg daily. Preferably, valsartan is applied twice a day with a dose of 80 mg or 160 mg, respectively, each. Corresponding doses may be taken, for example, in the morning, at mid-day or in the evening.

In case of HMG-Co-A reductase inhibitors , preferred dosage unit forms of HMG-Co-A reductase inhibitors are, for example, tablets or capsules comprising e.g. from about 5 mg to about 120 mg, preferably, when using fluvastatin, for example, 20 mg, 40 mg or 80 mg (equivalent to the free acid) of fluvastatin, for example, administered once a day.

In case of ACE inhibitors, preferred dosage unit forms of ACE inhibitors are, for example, tablets or capsules comprising e.g. from 3 to 40 mg, preferably from about 5 mg to about 20 mg, preferably 5 mg, 10 mg, 20 mg or 40 mg, of benazepril; from about 6.5 mg to 100 mg, preferably 6.25 mg, 12.5 mg, 25 mg, 50 mg, 75 mg or 100 mg, of captopril; from about 2.5 mg to about 20 mg, preferably 2.5 mg, 5 mg, 10 mg or 20 mg, of enalapril; from about 10 mg to about 20 mg, preferably 10 mg or 20 mg, of fosinopril; from about 2.5 mg to about 4 mg, preferably 2 mg or 4 mg, of perindopril; from about 5 mg to about 20 mg, preferably 5 mg, 10 mg or 20 mg, of quinapril; or from about 1.25 mg to about 5 mg, preferably 1.25 mg, 2.5 mg, or 5 mg, of ramipril. Preferred is t.i.d. administration.

Pharmaceutical preparations for enteral and parenteral administration are, for example, those in dosage unit forms, such as dragées, tablets or capsules and also ampoules. They are prepared in a manner known *per* se, for example by means of conventional mixing, granulating, confectioning, dissolving or lyophilising processes. For example, pharmaceutical preparations for oral administration can be obtained by combining the active ingredient with solid carriers, where appropriate granulating a resulting mixture, and processing the mixture or granulate, if desired or necessary after the addition of suitable adjuncts, into tablets or dragée cores.

Other orally administrable pharmaceutical preparations are dry-filled capsules made of gelatin, and also soft, sealed capsules made of gelatin and a plasticiser, such as glycerol or sorbitol. The dry-filled capsules may contain the active ingredient in the form of a granulate, for example in admixture with fillers, such as lactose, binders, such as starches, and/or glidants, such as talc or magnesium stearate, and, where appropriate, stabilisers. In soft capsules the active ingredient is preferably dissolved or suspended in suitable liquids, such as fatty oils, paraffin oil or liquid polyethylene glycols, it being possible also for stabilisers to be added.

Parenteral formulations are especially injectable fluids that are effective in various manners, such as intravenously, intramuscularly, intraperitoneally, intranasally, intradermally or subcutaneously. Such fluids are preferably isotonic aqueous solutions or suspensions which can be prepared before use, for example from lyophilised preparations which contain the active ingredient alone or together with a pharmaceutically acceptable carrier. The pharmaceutical preparations may be sterilised and/or contain adjuncts, for example preservatives, stabilisers, wetting agents and/or emulsifiers, solubilisers, salts for regulating the osmotic pressure and/or buffers.

Suitable formulations for transdermal application include an effective amount of the active ingredient with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the active ingredient of the skin of the host at a controlled and predetermined rate over a prolonged period of time, and means to secure the device to the skin.

The combinations and compositions of the invention may be used for the treatment and/or prevention of hypertension (whether for malignant, essential, reno-vascular, diabetic, isolated systolic, or other secondary type of hypertension), heart failure such as diastolic and congestive heart failure (acute and chronic), left ventricular dysfunction, endothelial dysfunction, diastolic dysfunction, hypertrophic cardiomyopathy, diabetic cardiac myopathy, supraventricular and ventricular arrhythmias, atrial fibrillation (AF), cardiac fibrosis, atrial flutter, detrimental vascular remodeling, plaque stabilization, myocardial infarction (MI) and its sequelae, atherosclerosis including coronary arterial disease (CAD), angina pectoris (whether unstable or stable), renal insufficiency (diabetic and non- diabetic), renal fibrosis, polycystic kidney disease (PKD), type 2 diabetes, metabolic syndrome, secondary aldosteronism, primary and secondary pulmonary hypertension, renal failure conditions such as nephrotic syndrome, diabetic nephropathy, glomerulonephritis, scleroderma, glomerular sclerosis, proteinuria of primary renal disease, renal vascular hypertension, diabetic retinopathy and end-stage renal disease (ESRD), the management of other vascular disorders such as migraine, peripheral vascular disease (PVD), Raynaud's disease, luminal hyperplasia, cognitive dysfunction (such as Alzheimer's), glaucoma and cerebrovascular disease such as embolic or thrombotic stroke.

Alternatively or additionally, the combinations and compositions of the invention may be used to treat or prevent a COX-2 dependent disorder. The term is understood to mean any disease or condition in which COX-2 is implicated, for example inflammation, pyresis, pain, osteoarthritis, rheumatoid arthritis, dysmenorrhea, migraine headache, cancer (such as of the digestive tract, e.g., colon cancer and melanoma), cancer pain, acute pain, chronic pain, neurodegenerative diseases (such as multiple sclerosis, Parkinson's disease and Alzheimer's disease), cardiovascular disorders (such as atherosclerosis, coronary artery disease and arteriosclerosis), osteoporosis, gout, acute gout, asthma, lupus and psoriasis.

The term "hypertension" or "hypertensive" refers to a condition where the pressure of blood within the blood vessels is higher than normal as it circulates through the body. When the systolic pressure exceeds 150 mmHg or the diastolic pressure exceeds 90 mmHg for a sustained period of time, damage is done to the body. For example, excessive systolic pressure can rupture blood vessels anywhere, and when it occurs within the brain, a stroke results. Hypertension may also cause thickening and narrowing of the blood vessels which ultimately could lead to atherosclerosis.

The term "severe hypertension" refers to hypertension characterized by a systolic blood pressure of ≥ 180 mmHg and a diastolic blood pressure of ≥ 110 mmHg.

The term "pulmonary hypertension" (PH) refers to a blood vessel disorder of the lung in which the pressure in the pulmonary artery rises above normal level of ≤ 25/10 (especially primary and secondary PH), e.g., because the small vessels that supply blood to the lungs constrict or tighten up. According to the WHO, PH may be divided into five categories: pulmonary arterial hypertension (PAH), a PH occurring in the absence of a known cause is referred to as primary pulmonary hypertension, while secondary PH is caused by a condition selected, e.g., from emphysema; bronchitis; collagen vascular diseases, such as scleroderma, Crest syndrome or systemic lupus erythematosus (SLE); PH associated with disorders of the respiratory system; PH due to chronic thrombotic or embolic disease; PH due to disorders directly affecting the pulmonary blood vessels; and pulmonary venous hypertension (PVH).

The term "malignant hypertension" is usually defined as very high blood pressure with swelling of the optic nerve behind the eye, called papilledema (grade IV Keith-Wagner hypertensive retinopathy). This also includes malignant HTN of childhood.

The term "isolated systolic hypertension" refers to hypertension characterized by a systolic blood pressure of ≥ 140 mmHg and a diastolic blood pressure of < 90 mmHg.

The term "familial dyslipidemic hypertension" is characterized by mixed dyslipidemic disorders. Biomarkers include oxidized LDL, HDL, glutathione and homocysteine LPa.

The term "renovascular hypertension" (renal artery stenosis) refers to a condition where the narrowing of the renal artery is significant which leads to an increase of the blood pressure resulting from signals sent out by the kidneys. Biomarkers include renin, PRA and prorenin.

The following examples are intended to illustrate the invention and are not to be construed as being limitations thereon.

### EXAMPLES

### A. Formulation Examples

### Example 1

**Table 1**

| **Ingredient** | **Amount per 200 mg tablet batch (kg)** |
|---|---|
| **Core** | |
| **Granulation** | |
| 5-methyl-2-(2'-chloro-6'- | 50** |
| fluoroanilino)phenylacetic acid drug | |
| substance | |
| Microcrystalline cellulose, NF (PH 101) | 12.85 |
| Lactose monohydrate, NF | 11.65 |
| Croscarmellose sodium, NF | 1 |
| Povidone, USP | 4 |
| Titanium dioxide, USP | 2 |
| Water, purified ***, USP | 20.375 |

| **Extra-granular Phase** | |
|---|---|
| Microcrystalline cellulose, NF (PH | 13 |
| 102) | |
| Croscarmellose sodium, NF | 3 |
| Titanium dioxide, USP | 2 |
| Magnesium stearate, NF | 0.5 |

| **Coating** | |
|---|---|
| Opadry white | 2.801 **** |
| Opadry yellow | 2.0 **** |
| Opadry red | 0.4 **** |
| Opadry black | 0.0504 **** |
| Water, purified ***, USP | 29.758 **** |

| | |
|---|---|
| ** The weight of drug substance is taken with reference to the dried substance (100 per cent) on the basis of the assay value (factorization). The difference in weight is adjusted by the amount of microcrystalline cellulose used. *** Removed during processing. **** Includes a 50 % excess for loss during the coating process. | |

Table 1, above, sets out the formula for a batch of approximately 250,000 immediate release film-coated tablets of 5-methyl-2-(2'-chloro-6'-fluoroanilino)-phenylacetic acid. To make the tablets, titanium dioxide is dispersed in water, followed by the addition of povidone and mixing for 20 minutes to make a povidone/titanium dioxide suspension. The drug substance, lactose, microcrystalline cellulose, and croscarmellose are mixed in a high shear mixer (e.g., a Collette Gral) for 5 minutes to form a drug mixture. The drug mixture is granulated in the high shear mixer with the povidone/titanium dioxide suspension. The suspension is pumped at a rate of 3 kg/min into the drug mixture. The resulting mixture is mixed an additional 90 seconds after all the suspension is added. The wet granulation is dried in a fluid bed dryer, using an inlet air temperature of 50 °C. The residual water target is 3.5 % (with a permissible range of 2.5 - 4.5 %). The dried granulation is passed through a screen using a mill (oscillator) and a 30 mesh screen. The previous steps are repeated to make a second granulation.

The extra-granular phase titanium dioxide is passed through a 60 mesh hand screen. The dry granulations are mixed with the extra-granular phase microcrystalline cellulose, croscarmellose sodium and titanium dioxide in a twin shell mixer for 300 revolutions to form a penultimate mixture. Magnesium stearate is passed through a 60 mesh hand screen and is mixed with the penultimate mixture in a twin shell mixer for 50 revolutions to form a tableting mixture. The tableting mixture is pressed into tablets using a tablet press and oval punches.

The coating powders (Opadry) are mixed with purified water to make a 15 % w/w coating suspension. The tablets are film coated with the coating suspension in a coating pan using 60 °C to 75 °C inlet air temperature.

Table 2 sets out the contents of a 200 mg 5-methyl-2-(2'-chloro-6'-fluoroanilino)phenylacetic acid film-coated tablet.

**Table 2**

| **Ingredient** | **Theoretical** | **Function** |
|---|---|---|
| | **amount [mg]** | |
| **Core** | | |
| 5-methyl-2-(2'-chloro-6'- | 200 | Active |
| fluoroanilino)phenylacetic acid | | substance |
| drug substance | | |
| Microcrystalline cellulose (PH | 51.4 | Filler |
| 101) | | |
| Lactose | 46.6 | Filler |
| Povidone | 16 | Binder |
| Titanium dioxide | 8 | Color |
| Croscarmellose sodium | 4 | Disintegrant |
| Water, purified ^{*} | Q.S. | Granulating |
| | | liquid |
| **Extragranular phase** | | |
| Microcrystalline cellulose (PH | 52 | Filler |
| 102) | | |
| Croscarmellose sodium | 12 | Disintegrant |
| Titanium dioxide | 8 | Color |
| Magnesium stearate | 2 | Lubricant |
| Core weight | **400** | |
| **Coating** | | |
| Opadry white (OOF18296) | 7.4676 | Color |
| Opadry yellow (OOF12951) | 5.3312 | Color |
| Opadry red (OOF15613) | 1.0668 | Color |
| Opadry black (00F17713) | 0.1344 | Color |
| Water, purified * | Q.S. | Coating solvent |
| **Total weight** | **414** | |

| | | |
|---|---|---|
| * removed during processing | | |

In addition, the tablet formulations may contain 5-methyl-2-(2'-chloro-6'-fluoroanilino)benzyl alcohol and/or 5-methyl-2-(2'-chloro-6'-fluoroanilino)benzoic acid in an amount between about 0.01 and 2% by weight, more specifically between about 0.1 and 1%.

### Example 2

An alternative formulation is as set out in Table 3, with information about as percentage w/w, mg/dose, and kg/ 50,000 tablet batch.

**(a) Table 3 Alternative formulation composition**

| **% w/w** | **Ingredient** | **Mg/dose** | **Kg/batch** |
|---|---|---|---|
| | Granulation | | |
| 65.04 | 5-methyl-2-(2'-chloro-6'-fluoroanilino) | 400.00 | 20.00 |
| | phenylacetic acid drug substance | | |
| 2.15 | Croscarmellose sodium, NF (Ac-Di-Sol) | 13.22 | 0.661 |
| 6.60 | Povidone K30, USP | 40.59 | 2.029 |
| 18.12 | Purified water, USP* | Qs | Qs |
| | Blending | | |
| 23.56 | Microcrystalline Cellulose, NF (Avicel PH | 144.90 | 6.066 |
| | 102) | | |
| 2.15 | Croscarmellose sodium, NF (Ac-Di-Sol) | 13.22 | 0.553 |
| 0.50 | Magnesium Stearate, NF (vegetable source) | 3.07 | 0.128 |
| | Film Coating | | |
| 84.46 | Opadry, Global White 00F18296 | 15.2028 | 0.296637 |
| 14.03 | Opadry, Global Red 00F15613 | 2.5254 | 0.049275 |
| 1.51 | Opadry, Global Black 00F17713 | 0.2718 | 0.005303 |
| | Purified Water, USP* | Qs | 1.990218 |
| | Film Coated Tablet Weight | 633.00 | |

| | | | |
|---|---|---|---|
| *Does not appear in final product. Percentage of water added used for granulation based on the dry weight of drug substance and croscarmellose sodium. | | | |

The batch is granulated as described in Example 1. The granulation is dried to residual moisture (% LOD) of 1.79%. The formulation process is the same as for the development batches as described above, except for the additional step of coating with Opadry in a coating pan. The coating powders (Opadry) are mixed with purified water to make a 15 % w/w coating suspension. The tablets are film coated with the coating suspension in a coating pan using 60°C to 75°C inlet air temperature. Based on friability data, a target force of 18 KN (16 - 20 KN range) is used to compress the remainder of the batch, resulting in acceptable friability (less than 0.5%) and the disintegration times of less than 5 mins. The ejection force is approximately 800 N throughout the compression run. This demonstrates that the blend is lubricated adequately. No picking/sticking is observed on the punch surfaces after 225 minutes. Thus, a smaller size tablet with high drug loading (65%) is achieved using a high shear granulation process, using 17 X 6.7 mm ovaloid tooling to get tablets with acceptable hardness and friability characteristics.
In addition, the tablet formulations may contain 5-methyl-2-(2'-chloro-6'-fluoroanilino)benzyl alcohol and/or 5-methyl-2-(2'-chloro-6'-fluoroanilino)benzoic acid in an amount between about 0.01 and 2% by weight, more specifically between about 0.1 and 1%.

### Example 3

Wet granulated tablet composition

| Amount per tablet | | Ingredient |
|---|---|---|
| 25 | mg | COX-2 inhibitor |
| 79.7 | mg | Microcrystalline cellulose |
| 79.7 | mg | Lactose monohydrate |
| 6 | mg | Hydroxypropyl cellulose |
| 8 | mg | Croscarmellose sodium |
| 0.6 | mg | Iron oxide |
| 1 | mg | Magnesium stearate |

Tablet dose strengths of between 5 and 125 mg can be accomodated by varying total weight, and the ratio of the first three ingredients. Generally it is preferable to maintain a 1:1 ratio for microcrystalline cellulose: lactose monohydrate.

### Example 4

Hard gelatine capsule composition

| Amount per capsule | | Ingredient |
|---|---|---|
| 25 | mg | COX-2 inhibitor |
| 37 | mg | Microcrystalline cellulose |
| 37 | mg | Lactose anhydrate |
| 1 | mg | Magnesium stearate |
| 1 | capsule | Hard gelatin capsule |

Capsule dose strengths of between 1 and 50 mg can be accomodated by varying total fill weight, and the ratio of the first three ingredients. Generally it is preferable to maintain a 1:1 ratio for microcrystalline cellulose:lactose monohydrate.

### Example 5

Oral solution

| Amount per 5mL | | Ingredient |
|---|---|---|
| 50 | mg | COX-2 inhibitor |
| to 5 mL with Polyethylene oxide 400 | | |

### Example 6

### Intravenous infusion

| Amount per 200 mL dose | | Ingredient |
|---|---|---|
| 1 | mg | COX-2 inhibitor |
| 0.2 | mg | Polyethylene oxide 400 |
| 1.8 | mg | Sodium chloride |
| to 200 | mL | Purified water |

### Example 8: Treatment of Patients

**Method:** A 4-week, multicenter, randomized, double-blind, double-dummy, parallel group study of hypertensive OA patients aged ≥50 years (office BP ≤140/90 mmHg) on stable antihypertensive treatment. Eligible patients were randomized to lumiracoxib 100 mg od or ibuprofen 600 mg tid (1:1). The primary outcome measure was the change from baseline (BL) at Week 4 in 24-hour mean ambulatory systolic BP (MASBP) in the intent-to-treat (ITT) population using ANCOVA. The secondary analyses included 24-hour mean ambulatory diastolic BP (MADBP clinically relevant increase in ABP. In addition, a post-hoc subgroup analysis was performed to evaluate the primary and secondary analysis at Week 4 (using ANCOVA) for patients exclusively on ACE inhibitors as antihypertensive drug.

**Results:** Of the 787 patients randomized (n=394 lumiracoxib, n=393 ibuprofen), 741 patients (94%) completed the study. Change from BL at Week 4 in 24-hour MASBP showed an estimated difference of 5 mmHg in favour of lumiracoxib (95% Cl: -6.1, -3.8, p<0.001). Patients on lumiracoxib showed a decrease in MASBP of 2.7 mmHg compared with a 2.2 mmHg increase in patients taking ibuprofen. Similar results were obtained for 24-hour MADBP (estimated treatment difference: -2 mmHg [95% Cl: -2.7, -1.3], p<0.001).
The effect of lumiracoxib on BP profile as compared to ibuprofen was more pronounced in patients using ACE-inhibitors (table)

**Table: Change from baseline in 24 h MASBP and MADBP with lumiracoxib (n=140) compared to ibuprofen (n=132) for patients exclusively on ACE- inhibitors**

| | Lumiracoxib vs. Ibuprofen |
|---|---|
| **24 h MASBP** | |
| Difference in LSM (95% CI) | -7.5 (-9.6, -5.5)* |
| **24 h MADBP** | |
| Difference in LSM (95% CI) | -3.9 (-5.1,-2.7)* |

| | |
|---|---|
| *p<0.001 Clinically relevant increase (defined as >= 5 mm Hg increase in 24 h MADBP and/or >= 10 mm Hg increase in 24h MASBP) was observed in 13 (9.3%) patients treated with lumiracoxib as compared to 39 (29.5%) patients treated with ibuprofen ( p< 0.001). | |

Conclusion: Lumiracoxib 100 mg od maintained superior BP profile over ibuprofen 600 mg tid in hypertensive OA patients treated with ACE-inhibitors.

## Claims

1. A combination comprising a COX-2 inhibitor and an antihypertensive for the treatment of a COX-2 dependent disorder in a hypertensive patient.

2. A pharmaceutical composition for the treatment of a COX-2 dependent disorder, the composition comprising a COX-2 inhibitor and an antihypertensive.

3. A pharmaceutical composition for the treatment of pain in a hypertensive patient with osteoarthritis, the composition comprising a COX-2 inhibitor and an antihypertensive.

4. Use of a COX-2 inhibitor for the preparation of a medicament for use in combination with an antihypertensive for treatment of pain in a hypertensive patient.

5. Use of a COX-2 inhibitor in the manufacture of a medicament for the treatment of pain in a patient with osteoarthritis and controlled hypertension.

6. A method of treating a patient suffering from pain comprising ad ministering to the patient an effective amount of a COX-2 inhibitor and an antihypertensive, the COX-2 inhibitor and antihypertensive being administered simultaneously, separately or sequentially;

7. A method of treating or preventing hypertension in a patient in need thereof comprising administering to the patient an effective amount of a COX-2 inhibitor and an antihypertensive, the COX-2 inhibitor and antihypertensive being administered simultaneously, separately or sequentially.

8. A method of reducing blood pressure in a hypertensive patient comprising administering to the patient an effective amount of a COX-2 inhibitor and an antihypertensive, the COX-2 inhibitor and antihypertensive being administered simultaneously, separately or sequentially.

9. A package comprising a COX-2 inhibitor together with instructions for use in combination with an antihypertensive for treatment of a COX-2 dependent disorder in a patient suffering from hypertension.

10. A package comprising a COX-2 inhibitor and an antihypertensive together with instructions for use for treatment of pain in a patient suffering from hypertension.

11. A combination according to claim 1 wherein the COX-2 inhibitor is lumiracoxib.

12. A combination according to claim 1 or 11 wherein the antihypertensive is an ACE inhibitor.

13. A composition according to claim 2 or 3 wherein the COX-2 inhibitor is lumiracoxib.

14. A composition according to claim 2 or 3 wherein the antihypertensive is an ACE inhibitor.

15. Use according to claim 4 or 5 wherein the COX-2 inhibitor is lumiracoxib.

16. Use according to claim 4 or 15 wherein the antihypertensive is an ACE inhibitor.

17. A method according to any one of claims 6-8 wherein the COX-2 inhibitor is lumiracoxib.

18. A method according to any one of claims 6-8 or 17 wherein the antihypertensive is an ACE inhibitor.

19. A package according to claim 9 or 10 wherein wherein the COX-2 inhibitor is lumiracoxib.

20. A package according to claim 9, 10 or 19 wherein wherein the antihypertensive is an ACE inhibitor.
